# EUROPÄISCHE PATENTSCHRIFT

(11) **EP 1 265 904 B1**
(45) Veröffentlichungstag und Bekanntmachung des Hinweises auf die Patenterteilung: **22.09.2004**
(21) Anmeldenummer: 01911553.4
(22) Anmeldetag: 30.01.2001
(51) Int. Cl.: C07D 513/06, A61K 31/54, A61P 25/00

(54) **NEUE POSITIVE ALLOSTERISCHE AMPA-REZEPTOR MODULATOREN (PAARM), VERFAHREN ZU DEREN HERSTELLUNG UND DEREN VERWENDUNG ALS ARZNEIMITTEL**
NOVEL POSITIVE ALLOSTERIC AMPA RECEPTOR MODULATORS (PAARM), METHOD FOR THE PRODUCTION AND USE THEREOF AS MEDICAMENTS
NOUVEAUX MODULATEURS (PAARM) ALLOSTERIQUES POSITIFS DE RECEPTEURS AMPA, LEUR METHODE DE PRODUCTION ET LEUR UTILISATION COMME MEDICAMENTS

(30) Priorität: 02.02.2000 DE 10004572
(43) Veröffentlichungstag der Anmeldung: 18.12.2002
(73) Patentinhaber: Boehringer Ingelheim Pharma GmbH & Co.KG, 55218 Ingelheim am Rhein (DE)
(72) Erfinder: WINTER, Karin, 55435 Gau-Algesheim (DE); WEISER, Thomas, 55268 Nieder-Olm (DE); BLECH, Stefen, Matthias, 88447 Warthausen (DE); CECI, Angelo, 88400 Biberach (DE)
(86) Internationale Anmeldenummer: PCT/EP2001/000960
(87) Internationale Veröffentlichungsnummer: WO 2001/057045

(56) Entgegenhaltungen:
- EP-A- 0 430 114
- WO-A-93/21170
- WO-A-98/12185
- WO-A-99/42456
- US-A- 4 895 863
- US-A- 5 688 803
- US-A- 5 985 871
- US-A- 6 096 744

## Beschreibung

Die vorliegende Erfindung betrifft neue, positive allosterische AMPA-Rezeptor Modulatoren, Verfahren zu deren Herstellung sowie deren Verwendung als Arzneimittel.

Benzothiadiazide sind im Stand der Technik als allosterische AMPA-Rezeptor Modulatoren bekannt (WO 98/12185, WO 93/21170, US 5,985,871). Ebenso wurden Benzoxazine mit einer solchen Wirkung beschrieben (WO 99/ 42456).

Bei den erfindungsgemäßen Verbindungen handelt es sich um Verbindungen der allgemeinen Formel (I) 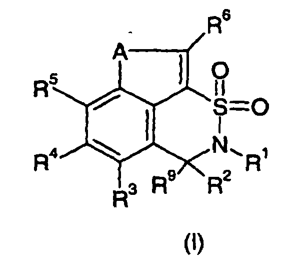 worin
A ein Schwefelatom, Sauerstoffatom, NH oder N-C₁-C₄-Alkyl,
R¹ ein Rest ausgewählt aus der Gruppe bestehend aus Wasserstoff, einer gegebenenfalls durch ein oder mehrere Halogenatome substituierten C₁-C₆-Alkylgruppe, -SO₂H, -SO₂-C₁-C₆-Alkyl, -SO-C₁-C₆-Alkyl, -CO-C₁-C₆-Alkyl, -O, Phenyl-C₁-C₄-Alkyl, -C₁-C₄-Alkyl-NR⁷R⁸ und -C₁-C₄-Alkyl-O- C₁-C₄-Alkyl, C₃-C₆-Cycloalkyl,
R², R⁹ gleich oder verschieden, ein Rest ausgewählt aus der Gruppe bestehend aus Wasserstoff, einer gegebenenfalls durch ein oder mehrere Halogenatome substituierten C₁-C₆-Alkylgruppe, Halogen, -NO₂, -SO₂H, -SO₂-C₁-C₆-Alkyl, -SO-C₁-C₆-Alkyl, -CO-C₁-C₆-Alkyl, -OH, -O-C₁-C₆-Alkyl, -S-C₁-C₆-Alkyl, -C₁-C₄-Alkyl-NR⁷R⁸ und -C₁-C₄-Alkyl-O- C₁-C₄-Alkyl, C₃-C₆-Cycloalkyl, oder
R¹ und R² gemeinsam eine C₂-C₆-Alkylenbrücke,
R⁷, R⁸ gleich oder verschieden, Wasserstoff oder C₁-C₄-Alkyl, und
R³, R⁴, R⁵, R⁶ gleich oder verschieden, ein Rest ausgewählt aus der Gruppe bestehend aus Wasserstoff, einer gegebenenfalls durch ein oder mehrere Halogenatome substituierten C₁-C₆-Alkyl Gruppe, Phenyl-C₁-C₄-Alkyl, Halogen, -CN, -NO₂, -SO₂H, -SO₃H, -SO₂-C₁-C₆-Alkyl, -SO-C₁-C₆-Alkyl, -SO₂-NR⁷R⁸, -COOH, -CO-C₁-C₆-Alkyl,, -O-CO-C₁-C₄-Alkyl, -CO-O-C₁-C₄-Alkyl, -CO-C₁-C₄-Alkyl, -OH, -O-C₁-C₆-Alkyl, -S-C₁-C₆-Alkyl, -NR⁷R⁸ und einem gegebenenfalls einfach oder mehrfach durch Halogenatome, -NO₂, -SO₂H oder C₁-C₄-Alkyl substituierten Aryl-Rest, bedeuten,
gegebenenfalls in Form ihrer verschiedenen Enantiomeren und .
Diastereomeren, sowie deren pharmakologisch unbedenklichen Salze.

Bevorzugt sind Verbindungen der allgemeinen Formel (I), worin
A ein Schwefelatom, Sauerstoffatom oder N-C₁-C₂―Alkyl,
R¹ ein Rest ausgewählt aus der Gruppe bestehend aus Wasserstoff, einer gegebenenfalls durch ein oder mehrere Halogenatome substituierten C₁-C₆-Alkylgruppe, -SO₂H, -SO₂-C₁-C₆-Alkyl, -SO-C₁-C₆-Alkyl, -CO-C₁-C₆-Alkyl, -O, -C₁-C₄-Alkyl-NR⁷R⁸ und -C₁-C₄-Alkyl-O- C₁-C₄-Alkyl, Benzyl,
R², R⁹ gleich oder verschieden, ein Rest ausgewählt aus der Gruppe bestehend aus Wasserstoff, einer gegebenenfalls durch ein oder mehrere Halogenatome substituierten C₁-C₆-Alkylgruppe, Halogen, -NO₂, -SO₂H, -SO₂-C₁-C₆-Alkyl, -SO-C₁-C₆-Alkyl, -CO-C₁-C₆-Alkyl, -OH, -O-C₁-C₆-Alkyl, -S-C₁-C₆-Alkyl, -C₁-C₄-Alkyl-NR⁷R⁸ und -C₁-C₄-Alkyl-O- C₁-C₄-Alkyl, oder R¹ und R² gemeinsam eine C₃-C₆-Alkylenbrücke, und
R³, R⁴, R⁵, R⁶ gleich oder verschieden, ein Rest ausgewählt aus der Gruppe bestehend aus Wasserstoff, einer gegebenenfalls durch ein oder mehrere Halogenatome substituierten C₁-C₄-Alkyl Gruppe, Phenyl-C₁-C₄-Alkyl, Halogen, -CN, -NO₂, -SO₂H, -SO₃H, -SO₂CH₃, -SOCH₃, -CO-C₁-C₄-Alkyl, -OH, -O-C₁-C₄-Alkyl und -S-C₁-C₄-Alkyl bedeuten,
gegebenenfalls in Form ihrer verschiedenen Enantiomeren und Diastereomeren, sowie deren pharmakologisch unbedenklichen Salze.

Besonders bevorzugt sind Verbindungen der allgemeinen Formel (I), worin
A ein Schwefelatom oder N-C₁-C₂―Alkyl,
R¹, R², R⁹ gleich oder verschieden, Wasserstoff, C₁-C₄-Alkyl, Benzyl oder
R¹ und R² gemeinsam eine C₃-C₄-Alkylenbrücke, und
R³, R⁴, R⁵, R⁶ gleich oder verschieden, ein Rest ausgewählt aus der Gruppe bestehend aus Wasserstoff, C₁-C₄-Alkyl, CF₃, NO₂, Benzyl, -SO₂-C₁-C₄-Alkyl, -SO₃H und Halogen, bevorzugt Fluor, Chlor, Brom, insbesondere bevorzugt Fluor oder Chlor bedeuten,
gegebenenfalls in Form ihrer verschiedenen Enantiomeren und

Diastereomeren, sowie deren pharmakologisch unbedenklichen Salze.

Weiterhin besonders bevorzugt sind Verbindungen der allgemeinen Formel (I), worin
A ein Schwefelatom oder N-CH₃,
R¹, R², R⁹ gleich oder verschieden, Wasserstoff, C₁-C₄-Alkyl oder
R¹ und R² gemeinsam eine C₃-C₄-Alkylenbrücke,
R³, R⁵, R⁶ gleich oder verschieden, ein Rest ausgewählt aus der Gruppe bestehend aus Wasserstoff, C₁-C₄-Alkyl und Halogen, bevorzugt Fluor, Chlor, Brom, insbesondere bevorzugt Fluor oder Chlor, und
R⁴ Wasserstoff, Halogen oder C₁-C₄-Alkyl bedeuten,
gegebenenfalls in Form ihrer verschiedenen Enantiomeren und

Diastereomeren, sowie deren pharmakologisch unbedenklichen Salze.

Erfindungsgemäß von besonderer Bedeutung sind die Verbindungen der allgemeinen Formel (I), worin
R¹ für Methyl, Ethyl, i-Propyl, n-Butyl oder Benzyl steht, gegebenenfalls in Form ihrer verschiedenen Enantiomeren und Diastereomeren, sowie deren pharmakologisch unbedenklichen Salze.

Insbesondere bevorzugt sind Verbindungen der allgemeinen Formel (I), worin
A ein Schwefelatom,
R¹ Methyl,
R², R⁹ Wasserstoff,
R³ ein Rest ausgewählt aus der Gruppe bestehend aus Wasserstoff, Methyl, CN und Halogen, bevorzugt Fluor, Chlor, Brom, insbesondere bevorzugt Fluor oder Chlor,
R⁵ ein Rest ausgewählt aus der Gruppe bestehend aus Wasserstoff, Methyl und Halogen, bevorzugt Fluor, Chlor, Brom, insbesondere bevorzugt Fluor oder Chlor,
R⁴ Wasserstoff, und
R⁶ Wasserstoff oder Methyl, bevorzugt Wasserstoff bedeuten,
gegebenenfalls in Form ihrer pharmakologisch unbedenklichen Salze.

Ferner besonders bevorzugt sind Verbindungen der allgemeinen Formel (I), worin
A ein Schwefelatom,
R¹ Methyl,
R³ Wasserstoff, Fluor oder Chlor, und
R^{2,} , R⁴, R⁵, R⁶ , R⁹ Wasserstoff bedeuten,
gegebenenfalls in Form ihrer pharmakologisch unbedenklichen Salze

Als Alkylgruppen werden, soweit nicht anders angegeben, verzweigte und unverzweigte Alkylgruppen mit 1 bis 6 Kohlenstoffatomen bevorzugt 1 bis 4 Kohlenstoffatomen bezeichnet. Beispielsweise werden genannt: Methyl, Ethyl, Propyl, Butyl, Pentyl und Hexyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl, Butyl, Pentyl und Hexyl alle denkbaren isomeren Formen der jeweiligen Reste. So umfaßt-beispielsweise Propyl, n-Propyl und iso-Propyl, Butyl umfaßt iso-Butyl, sec. Butyl und tert.-Butyl etc.

In den vorstehend genannten Alkylgruppen können gegebenenfalls ein oder mehrere Wasserstoffatome durch die Halogenatome Fluor, Chlor, Brom oder lod substituiert sein. Bevorzugt sind die Substituenten Fluor und Chlor. Besonders bevorzugt ist der Substituent Fluor. Es können gegebenenfalls auch alle Wasserstoffatome der Alkylgruppe ersetzt sein.

Die in der Gruppe Phenyl-C₁-C₄-Alkyl aufgeführte Alkylgruppe kann in verzweigter oder unverzweigter Form vorliegen. Soweit nicht anders angegeben sind Benzyl und Phenylethyl bevorzugte Phenyl-C₁-C₄-Alkyl Gruppen. Besonders bevorzugt ist Benzyl.

Als C₂-C₆-Alkylenbrücke werden, soweit nicht anders angegeben, verzweigte und unverzweigte Alkylengruppen mit 2 bis 6 Kohlenstoffatomen, beispielsweise Ethylen, Propylen, Methylethylen, Dimethylmethylen, n-Butylen, 1-Methylpropylen, 2-Methylpropylen, 1.1-Dimethylethylen, 1.2-Dimethylethylen etc. bezeichnet. Besonders bevorzugt sind n-Propylen- und n-Butylen-Brücken.

Als Arylrest wird ein aromatisches Ringsystem mit 6-10 Kohlenstoffatomen, vorzugsweise Phenyl bezeichnet.
In den vorstehend genannten Arylresten können gegebenenfalls ein oder mehrere Wasserstoffatome durch Halogenatome, -NO₂, -SO₂H oder -C₁-C₄-Alkyl, bevorzugt Fluor, Chlor, -NO₂, Ethyl oder Methyl, besonders bevorzugt Fluor oder Methyl substituiert sein.

Als C₃-C₆-Cycloalkyl werden gesättigte cyclische Kohlenwasserstoffreste mit 3 - 6 Kohlenstoffatomen beispielsweise Cyclopropyl, Cyclobutyl, Cyclopentyl oder Cyclohexyl bezeichnet.

Als Halogen wird, soweit nicht anders angegeben, Fluor, Chlor, Brom und lod, vorzugsweise Fluor, Chlor und Brom, besonders bevorzugt Fluor und Chlor, insbesondere bevorzugt Fluor bezeichnet.

Wie vorstehend genannt, können die Verbindungen der Formel (I) bzw. deren verschiedenen Enantiomere und Diastereomere, in ihre Salze, insbesondere für die pharmazeutische Anwendung, in ihre physiologisch und pharmakologisch verträglichen Salze überführt werden. Diese Salze können einerseits als physiologisch und pharmakologisch verträgliche Säureadditionssalze der Verbindungen der Formel (I) mit anorganischen oder organischen Säuren vorliegen. Andererseits kann die Verbindung der Formel (I) im Falle von R¹ gleich Wasserstoff durch Umsetzung mit anorganischen Basen auch in physiologisch und pharmakologisch verträgliche Salze mit Alkali- oder Erdalkalimetallkationen als Gegenion überführt werden. Zur Darstellung der Säureadditionssalze kommen beispielsweise Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden. Zur Darstellung der Alkali- und Erdalkalimetallsalze der Verbindung der Formel (I) in der R¹ Wasserstoff bedeutet, kommen vorzugsweise die Alkali- und Erdalkalihydroxide und -hydride in Betracht, wobei die Hydroxide und Hydride der Alkalimetalle, besonders des Natriums und Kaliums bevorzugt, Natrium- und Kaliumhydroxid besonders bevorzugt sind.

Die Herstellung der erfindungsgemäßen Verbindungen kann auf an sich bekannte Weise erfolgen. Folgende in Schema 1 und 2 dargestellte allgemeine Syntheseverfahren 1 und 2 sind als Erläuterung der Erfindung zu verstehen, ohne selbige auf deren Gegenstand zu beschränken.

### Verfahren 1

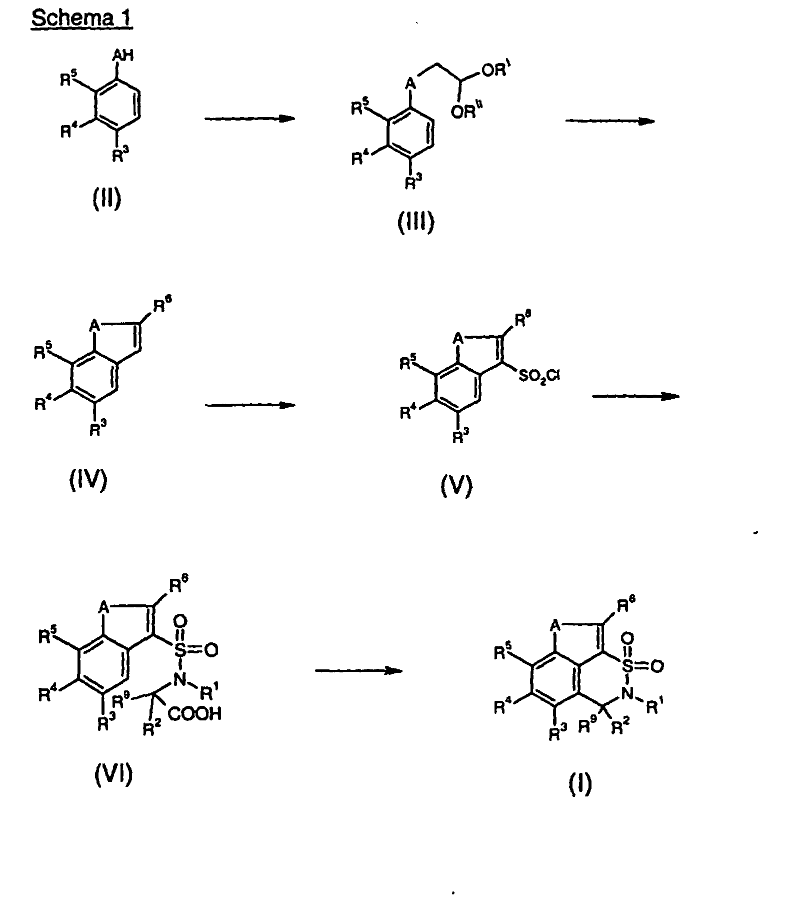

Ausgehend von einer Verbindung der Formel (IV) wird eine Verbindung der Formel (V) durch Sulfonierung und anschließende Chlorierung hergestellt. Die nach erfolgter Kondensation mit Aminoessigsäure-Derivaten erhaltene Verbindung der Formel (VI) wird durch Zusatz von Polyphosphorsäure zur Zielverbindung (I) zyklisiert. Nicht käufliche Verbindungen der Formel (IV) werden zuvor durch Überführung der Verbindungen der Formel (II) in die Verbindungen der Formel (III), worin R', R" gleich oder verschieden ,C1-C6-Alkyl oder gemeinsam eine 1,2-Ethylen oder 1,3-Propylen-Gruppe bedeuten, und deren anschließende Zyklisierung unter Einwirkung starker Säuren hergestellt.

Die allgemeine Herstellung der erfindungsgemäßen Verbindungen gemäß Schema 1 wird im folgenden anhand der Benzothiophenderivate (A=S) näher beschrieben. Das Verfahren läßt sich analog mit den entsprechenden Indol- bzw. Benzofuranderivaten durchführen:

### Synthese der Diethoxy-ethyl-thiophenole (III):

10 mmol des Thiophenols (II) werden in 2 - 100 ml, bevorzugt 3 - 20 ml , insbesondere bevorzugt 4 ml eines Alkohols, insbesondere Methanol, gelöst und mit 10 - 50 mmol, vorzugsweise 11 - 30 mmol, insbesondere bevorzugt 12 mmol einer Alkoholatlösung, insbesondere einer Natriumethanolatlösung versetzt. Nach 20 - 120 min, vorzugsweise 30 min, mit 10 - 50 mmmol, vorzugsweise 11 - 30 mmol, insbesondere bevorzugt 12 mmol, Bromacetaldehyddialkylacetal versetzt und die Lösung wird 2 - 16 h, vorzugsweise 5 h, auf 20 - 100 °C, vorzugsweise 50 - 70 °C, erwärmt. Nach Einengen der Lösung wird der Rückstand zwischen einem organischen Lösungsmittel und Wasser verteilt, insbesondere mit 30 ml Ether und 30 ml Wasser aufgenommen. Die Phasen werden getrennt und die wäßrige Phase wird anschließend mit Ether extrahiert. Die vereinigten organischen Extrakte werden über Natriumsulfat getrocknet und im Vakuum eingeengt. Das Rohprodukt wird ohne weitere Reinigung in die Folgereaktion eingesetzt.
Alternativ zum Lösungsmittel Methanol können Ethanol, Tetrahydrofuran, Toluol, Benzol, Dimethylformamid, Trichlormethan, Dichlormethan, Aceton oder Ethylacetat, alternativ zur Natriummethanolatlösung können Kaliumhydroxid, Kalium-tert.-Butylat, Lithiumhydroxid, Triethylamin, DBU (1,8-Diazabicyclo[5.4.0]undec-7-en), Natriumhydrid oder Kaliumcarbonat als Base eingesetzt werden.

### Synthese der Benzothiophene (IV):

Bei 140°C werden 10 - 100 ml, bevorzugt 30 ml, Polyphosphorsäure und 10 - 250 ml, bevorzugt 40 ml, Chlorbenzol vorgelegt und 10 mmol des Dialkoxy-ethylthiophenols (III) zugegeben. Nach 2 - 16 h, vorzugsweise 5 h, Rühren bei 90 - 160°C, bevorzugt 140°C werden die Phasen getrennt und die anorganische Phase wird mit Ether extrahiert. Die vereinigten organischen Extrakte werden über Na₂SO₄ getrocknet und im Vakuum eingeengt. Der Rückstand wird destillativ gereinigt. Alternativ zur Polyphosphorsäure können ein Gemisch aus Phoshorpentoxid/Phosphorsäure und Zinkchlorid, alternativ zu Chlorbenzol können Toluol oder Xylol eingesetzt werden.

### Sulfonierung der Benzothiophene (IV):

10 mmol des Benzothiophen-Derivates (IV) werden in 2 -100 ml, vorzugsweise 3 - 80 ml, insbesondere bevorzugt 4 ml, Essigsäureanhydrid gelöst und bei 0 - 50°C, vorzugsweise 5 - 20°C, 10 - 100 mmol, vorzugsweise 11 - 80 mmol, insbesondere bevorzugt 11 mmol, konz. Schwefelsäure zugetropft. Nach 2 - 16 h, vorzugsweise 5 h, Rühren bei 20 - 100°C, vorzugsweise 25 C°, wird das Gemisch auf eine gesättigte NaCl-Lösung gegossen. Die entstandenen Kristalle werden abgesaugt und getrocknet.
Alternativ zu Essigsäureanhydrid können Methylenchlorid, Diisopropylether, Ethylacetat, Trichlormethan, Toluol, Benzol oder 1,4-Dioxan, alternativ zu konz. Schwefelsäure können Oleum, Schwefeltrioxid, Chlorsulfate oder Kombinationen daraus, eingesetzt werden.

### Synthese der Benzothiophen-3-sulfonsäurechloride (V):

10 mmol der Benzothiophen-3-sulfonsäuren werden nacheinander mit 10 -500 mmol, vorzugsweise 90 mmol, Phosphoroxytrichlorid und 8 - 50 mmol, vorzugsweise 10 mmol Phosphorpentachlorid versetzt und 2 - 16 h, vorzugsweise 5 h, bei 20 - 100°C, vorzugsweise unter Rückfluss erhitzt. Anschließend wird das Reaktionsgemisch im Vakuum eingeengt und mit Eiswasser versetzt. Nach

Extraktion mit Ether werden die vereinten organischen Extrakte mit Dinatriumsulfat getrocknet und im Vakuum vom Lösungsmittel befreit. Das erhaltene Rohprodukt wird ohne Reinigung in die Folgestufen eingesetzt.
Alternativ zu dem Phosphoroxytrichlorid/Phosphorpentachlorid-Gemisch können Thionylchlorid, Phosphorpentachlorid, ein Phosphorsäure/Chlor-Gemisch oder Phosgen eingesetzt werden. Die Reaktion kann alternativ in den Verdünnungsmitteln Ethylacetat, Wasser, Acetonitril, N,N-Dimethylacetamid, Sulfolan, DMF, Hexan, oder Dichlorethan durchgeführt werden.

### Synthese der Benzothiophen-3-sulfonyl-amino-essigsäuren :

10 mmol der Chlorsulfonyl-benzothiophene, 10 - 100 mmol, vorzugsweise 11 - 30 mmol, insbesondere bevorzugt 12 mmol, der Aminoessigsäure und 10 - 100 mmol, vorzugsweise 11 - 30 mmol, insbesondere bevorzugt 12 mmol, Natriumhydroxid werden in 16 ml Wasser und 16 ml Toluol gelöst. Das Reaktionsgemisch wird 2 - 16 h bei 0 -110°C, vorzugsweise bei 65°C, gerührt, anschließend werden die Phasen getrennt. Die wäßrige Phase wird mit 2N Salzsäure sauer gestellt und mit Essigester extrahiert. Die vereinigten organischen Extrakte werden über Natriumsulfat getrocknet und im Vakuum eingeengt. Die Reinigung erfolgt durch Chromatographie. Alternativ zu Natriumhydroxid können Triethylamin, Kaliumcarbonat, Natriumhydrogencarbonat oder Natriumhydrid, alternativ zu Toluol können Tetrahydrofuran, Diethylether, Dichlormethan, Trichlormethan, Dioxan, Aceton, Benzol, Ethanol, Methanol, Ethylacetat oder Acetonitril eingesetzt werden.

### Zyklisierung der Benzothiophen-3-sulfonyl-amino-essigsäuren (VI):

10 mmol der Benzothiophen-3-sulfonyl-amino-essigsäuren werden mit 10 - 200 g, vorzugsweise 40 g, Polyphosphorsäure versetzt und 2 - 16 h, vorzugsweise 5 h, bei 20 - 110°C, vorzugsweise 75 - 95°C, gerührt. Anschließend wird das Reaktionsgemisch auf Eiswasser gegossen und mit Essigester extrahiert. Die vereinigten organischen Extrakte werden über Dinatriumsulfat getrocknet und eingeengt. Der Rückstand wird chromatographisch gereinigt.

### Verfahren 2

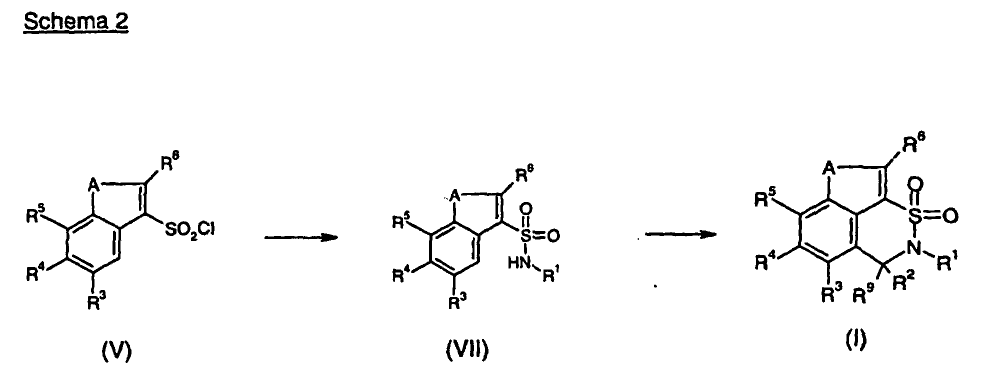

Die als Zwischenverbindungen in Verfahren 1 hergestellten Verbindungen der Formel (V) werden mit primären Aminen zu den Verbindungen der Formel (VII) umgesetzt und anschließend durch Zusatz einer Verbindung der Formel R²R⁹C=O in Gegenwart von starker Säure zu den Zielverbindungen (I) zyklisiert.
Zur Herstellung der Verbindungen der Formel (I), worin R¹ und R² Wasserstoff bedeuten, können Paraformaldehyd, Trioxan oder Formalin, als starke Säuren können Methansulfonsäure, Trifluoressigsäure, Schwefelsäure, Phosphorsäure oder Polyphosphorsäure eingesetzt werden.

Die allgemeine Herstellung der erfindungsgemäßen Verbindungen gemäß Schema 2 wird im folgenden anhand der Benzothiophenderivate (A=S) näher beschrieben. Das Verfahren läßt sich analog mit den entsprechenden Indol- bzw. Benzofuranderivaten durchführen.

### Synthese der Benzothiophen-sulfonamide(VII):

10 mmol der Chlor-benzothiophen-sulfonsäuren (V) werden mit einer alkoholischen Lösung des primären Amins (10 - 1000 mmol in 5 - 200 ml, beispielsweise 200 mmol in 50 ml Ethanol) versetzt und anschließend 2 - 16 h, vorzugsweise 5 h bei 0 - 100 C°, vorzugsweise unter Rückfluss, erhitzt. Anschließend wird das Reaktionsgemisch im Vakuum eingeengt und chromatographisch gereinigt.
Alternativ zum alkoholischen Lösungsmittel können Toluol, Benzol, Trichlormethan, Dichlormethan, Diethylether, Tetrahydrofuran, Wasser, Acetonitril, Essigsäureanhydrid, Aceton, Pyridin, Dimethylsulfoxid, Dimethylformamid, Dioxan oder Hexan eingesetzt werden.

### Zyklisierung der Benzothiophen-sulfonamide (VII) zu den Zielverbindungen (I):

10 mmol der Benzothiophen-sulfonamide werden in 0 - 100 ml, vorzugsweise 20 - 80 ml, insbesondere bevorzugt etwa 40 ml Methansulfonsäure gelöst und mit einer Lösung aus 3 - 50 mmol, vorzugsweise 4 - 30, insbesondere bevorzugt 5 mmol Trioxan in 0 - 100 ml, vorzugsweise etwa 12 ml, Trifluoressigsäure versetzt. Das Reaktionsgemisch wird 2 - 16 h, vorzugsweise 5 h, bei 20 - 100 C°, vorzugsweise 30 - 80 °C, insbesondere bevorzugt 35°C gerührt und anschließend auf Eiswasser gegossen. Nach Extraktion mit Ether und Trocknen der vereinigten organischen Extrakte über Na₂SO₄ wird die Lösung eingeengt. Das Rohprodukt wird chromatographisch gereinigt.
Alternativ zu Trioxan können Paraformaldehyd oder Formalin, alternativ zu Trifluoressigsäure können Bortrifluorid*Diethylether, Essigsäure, Polyphosphorsäure, Phosphorsäure oder Schwefelsäure eingesetzt werden. Als mögliche Lösungsmittel können Essigsäureanhydrid oder Dichlormethan verwendet werden.

Die neuen Verbindungen der allgemeinen Formel (I) können in Analogie zu nachfolgenden Synthesebeispielen synthetisiert werden. Diese Beispiele sind allerdings nur als exemplarische Vorgehensweise zur weitergehenden Erläuterung der Erfindung zu verstehen, ohne selbige auf dessen Gegenstand zu beschränken.

### Synthese - von 4-Methyl-4,5-dihydro-1,3-dithia-4-aza-acenaphthylen 3,3-dioxid (Beispiel 1)

### Stufe 1: Natrium-benzothiophen-3-sulfonat:

20 g Benzothiophen werden in 25 ml Essigsäureanhydrid vorgelegt, auf 5°C abgekühlt und langsam mit 8.7 ml konz. Schwefelsäure versetzt. Nach einer Reaktionszeit von 2 Stunden bei Raumtemperatur wird die Lösung auf 400 ml Eiswasser gegossen und anschließend mit 250 ml Diethylether gewaschen. Durch Zugabe von NaCl wird das Produkt aus der wäßrigen Lösung ausgesalzen, der ausfallende weiße Feststoff abgesaugt und im Trockenschrank bei 60°C getrocknet. Ausbeute: 26 g.

### Stufe 2: 3-Chlorsulfonyl-1-benzothiophen:

180 g Natrium-benzothiophen-3-sulfonat werden in 650 ml Phosphoroxychlorid vorgelegt und anschließend mit 156 g Phosphorpentachlorid versetzt. Die Reaktionsmischung wird 3.5 Stunden unter Rückfluß erwärmt, bevor das überschüssige Phosphoroxychlorid im Vakuum destillativ entfernt wird. Der Rückstand wird in 800 ml Chloroform aufgenommen und der entstandene Niederschlag durch Filtration abgetrennt. Das Filtrat wird eingeengt und in der Wärme in 800 ml Petrolether eingerührt. Die dabei ausfallende kristalline Substanz wird abfiltriert, mit Petrolether gewaschen und bei 35°C getrocknet.
Ausbeute: 113 g. Fp.: 88-89°C.

### Stufe 3: [(Benzothiophen-3-sulfonyl)-methyl-amino]-essigsäure:

Zu einem Gemisch aus 200 ml Toluol und 200 ml Wasser wird 6 g NaOH, 23.27 g 3-Chlorsulfonyl-benzothiophen und 13.36 g Sarcosin gefügt und 6.5 Stunden bei 60°C gerührt. Zur Aufarbeitung wird die wäßrige Phase abgetrennt und die organische Phase mit 100 ml 2 N NaOH-Lösung extrahiert. Die vereinigten wäßrigen Phasen werden mit konz. HCl angesäuert und anschließend zweimal mit je 300 ml Essigsäureethylester extrahiert. Nach Waschen mit gesättigter, wäßriger Kochsalzlösung und Trocknen über Magnesiumsulfat wird die organische Phase eingeengt. Das erhaltene Rohprodukt wird aus 100 ml Dichlorethan umkristallisiert. Ausbeute: 13.14 g. Fp.: 139-140°C.

### Stufe 4: 4,5-Dihydro-4-methyl-3,3-dioxid-thieno[2,3,4-ij][2,3]benzothiazin:

110 g Polyphosphorsäure werden mit 10.5 g [(Benzothiophen-3-sulfonyl)-methylamino]-essigsäure versetzt und 75 min bei 70-75°C gerührt, wobei eine starke Gasentwicklung auftritt. Das Reaktionsgemisch wird in 1 I warmes Wasser eingerührt und dreimal mit je 250 ml Methylenchlorid extrahiert. Die vereinigten organischen Phasen werden mit 300 ml einer 2 N NaOH-Lösung gewaschen und anschließend über Magnesiumsulfat getrocknet. Der nach dem Einengen verbleibende Rückstand wird chromatographisch gereinigt. Ausbeute: 3.8 g. Fp.: 149-150°C.

### Synthese von 4-Ethyl-4,5-dihydro-1,3-dithia-4-aza-acenaphthylen 3,3-dioxid (Beispiel 2):

1.90 g N-Ethyl-benzo[b]thiophen-3-sulfonamid werden in 25 ml Methansulfonsäure bei 35°C gelöst und mit einer Lösung aus 0.29 g Trioxan in 8 ml Trifluoressigsäure versetzt. Nach 2.5 h Rühren bei Raumtemperatur wird das Reaktionsgemisch auf 400 ml Eiswasser gegossen. Der entstandene Feststoff wird durch Filtration abgetrennt, mit 200 ml Wasser gewaschen und anschließend in 400 ml Ethanol/Isopropanol (1:1) heiß gelöst und durch emeute Filtration von festen Rückständen getrennt. Der nach dem Einengen des Filtrats verbleibende Rückstand wird chromatographisch gereinigt. Ausbeute: 0.82 g. Fp.: 156°C.

### Synthese von 8-Chlor-4-methyl-4,5-dihydro-1,3-dithia-4-aza-acenaphthylen 3,3-dioxid (Beispiel 3):

0.2 g 7-Chlor-benzo[b]thiophen-3-sulfonsäure-methylamid werden in 3 ml Methansulfonsäure bei 35°C gelöst und mit einer Lösung aus 0.03 g Trioxan in 0.9 ml Trifluoressigsäure versetzt. Nach 2 h Rühren bei 35°C wird das Reaktionsgemisch auf 100 ml Eiswasser gegossen und die wäßrige Phase wird mit Essigester extrahiert. Die gesammelten organischen Extrakte werden mit Na₂SO₄ getrocknet, im Vakuum eingeengt und anschließend chromatographisch gereinigt. Ausbeute: 0.06 g. Fp.: 146°C.

### Synthese von 6-Fluor-4-methyl-4,5-dihydro-1,3-dithia-4-aza-acenaphthylen 3,3-dioxid (Beispiel 4):

0.37 g 5-Fluor-benzo[b]thiophen-3-sulfonsäure-methylamid werden in 5.9 ml Methansulfonsäure bei 35°C gelöst und mit einer Lösung aus 0.06 g Trioxan in 1.8 ml Trifluoressigsäure versetzt. Nach 2.5 h Rühren bei 35°C wird das Reaktionsgemisch auf 100 ml Eiswasser gegossen und die wäßrige Phase wird mit Essigester extrahiert. Die gesammelten organischen Extrakte werden mit Na₂SO₄ getrocknet, im Vakuum eingeengt und anschließend chromatographisch gereinigt. Ausbeute: 0.22 g. Fp.: 175°C.

### Synthese von 6-Chlor-4-methyl-4,5-dihydro-1,3-dithia-4-aza-acenaphthylen 3,3-dioxid (Beispiel 5):

0.60 g 5-Chlor-benzo[b]thiophen-3-sulfonsäure-methylamid werden in 8.9 ml Methansulfonsäure bei 35°C gelöst und mit einer Lösung aus 0.09 g Trioxan in 2.7 ml Trifluoressigsäure versetzt. Nach 3 h Rühren bei 35°C wird das Reaktionsgemisch auf 100 ml Eiswasser gegossen und die wäßrige Phase wird mit Essigester extrahiert. Die gesammelten organischen Extrakte werden mit Na₂SO₄ getrocknet, im Vakuum eingeengt und anschließend chromatographisch gereinigt. Ausbeute: 0.30 g. Fp.: 196°C.

### Synthese von 1,4-Dimethyl-4,5-dihydro-1H-3-thia-1,4-diaza-acenaphthylene 3,3-dioxid (Beispiel 15):

4 g N,N'-Dimethylindol-3-sulfonamid werden in 100 ml Methansulfonsäure bei 35°C gelöst und mit 0.54 g Trioxan in 25 ml Trifluoressigsäure versetzt. Nach 1 h Rühren bei 35°C wird das Reaktionsgemisch auf Eis gegossen und die wäßrige Phase wird mit Essigester extrahiert. Die gesammelten organischen Extrakte werden mit Na₂SO₄ getrocknet, im Vakuum eingeengt und anschließend chromatographisch gereinigt. Ausbeute: 0.07 g. Fp.: 190°C.

### 4-Methyl-3,3-dioxo-4,5-dihydro-3H-1,3-dithia-4-aza-acenaphthylen-6-carbonitril (Beispiel 16)

Zu 0.32 g Kupfer(I)cyanid und 10 ml Pyridin werden 1 g 6-Brom-4-methyl-4,5-dihydro-1,3-dithia-4-aza-acenaphthylen 3,3-dioxid gegeben. Das Reaktionsgemisch wird 7 h bei 190°C erwärmt, anschließend auf 10 ml Ammoniak-Lösung gegossen und mit Wasser und Ether (je 30 ml) versetzt. Die wäßrige Phase wird mit Ether extrahiert. Die vereinten organischen Phasen werden mit 10 ml verdünnter Salzsäure gewaschen und anschließend über Na₂SO₄ getrocknet. Nach Einengen der Lösung im Vakuum folgt eine chromatographische Reinigung. Ausbeute: 0.06 g. Fp. 238°C.

Analog zu vorstehend beschriebener Vorgehensweise werden u.a. die folgenden Verbindungen der Formel IA, wobei Ph für Phenyl steht, erhalten:

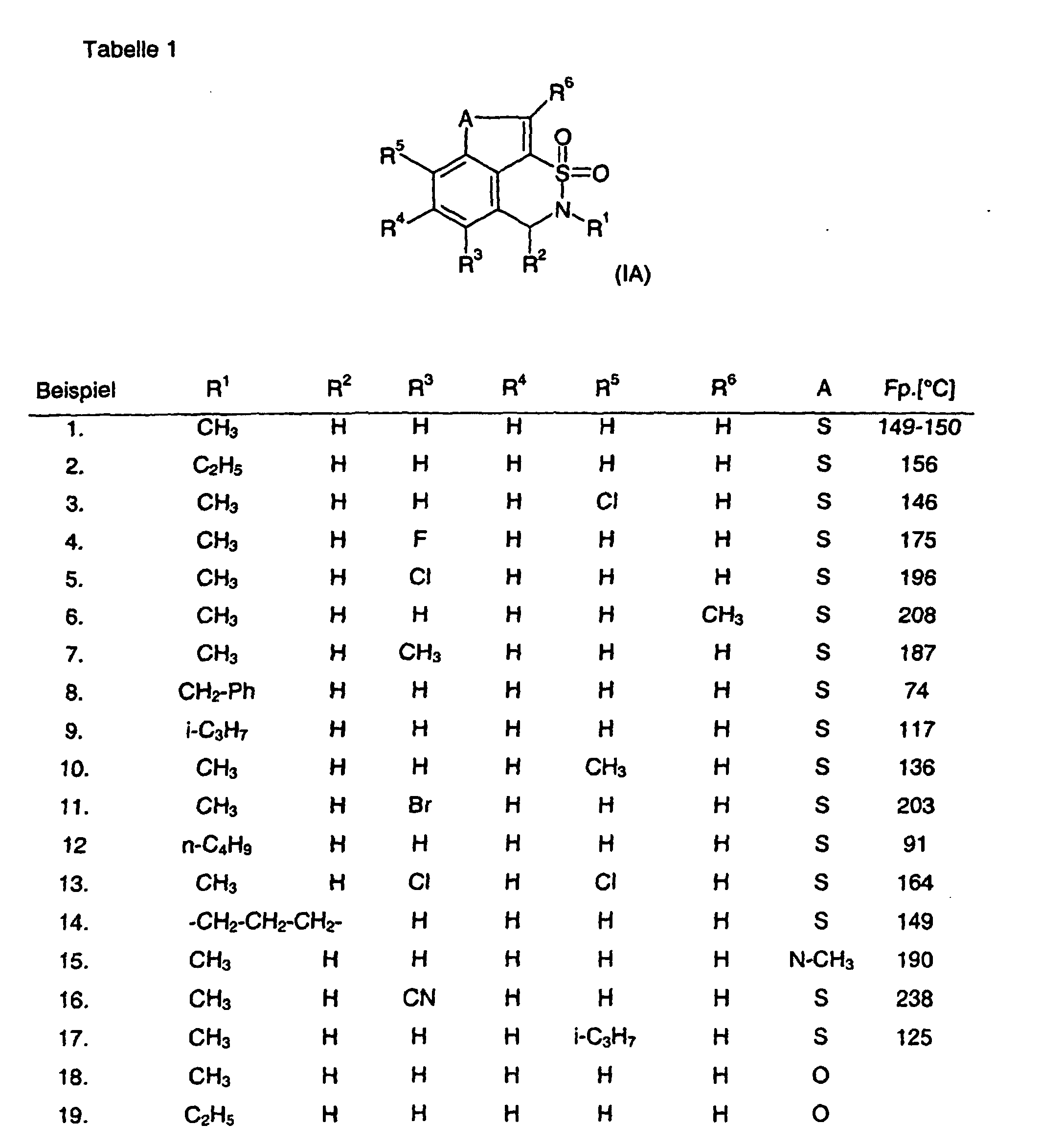

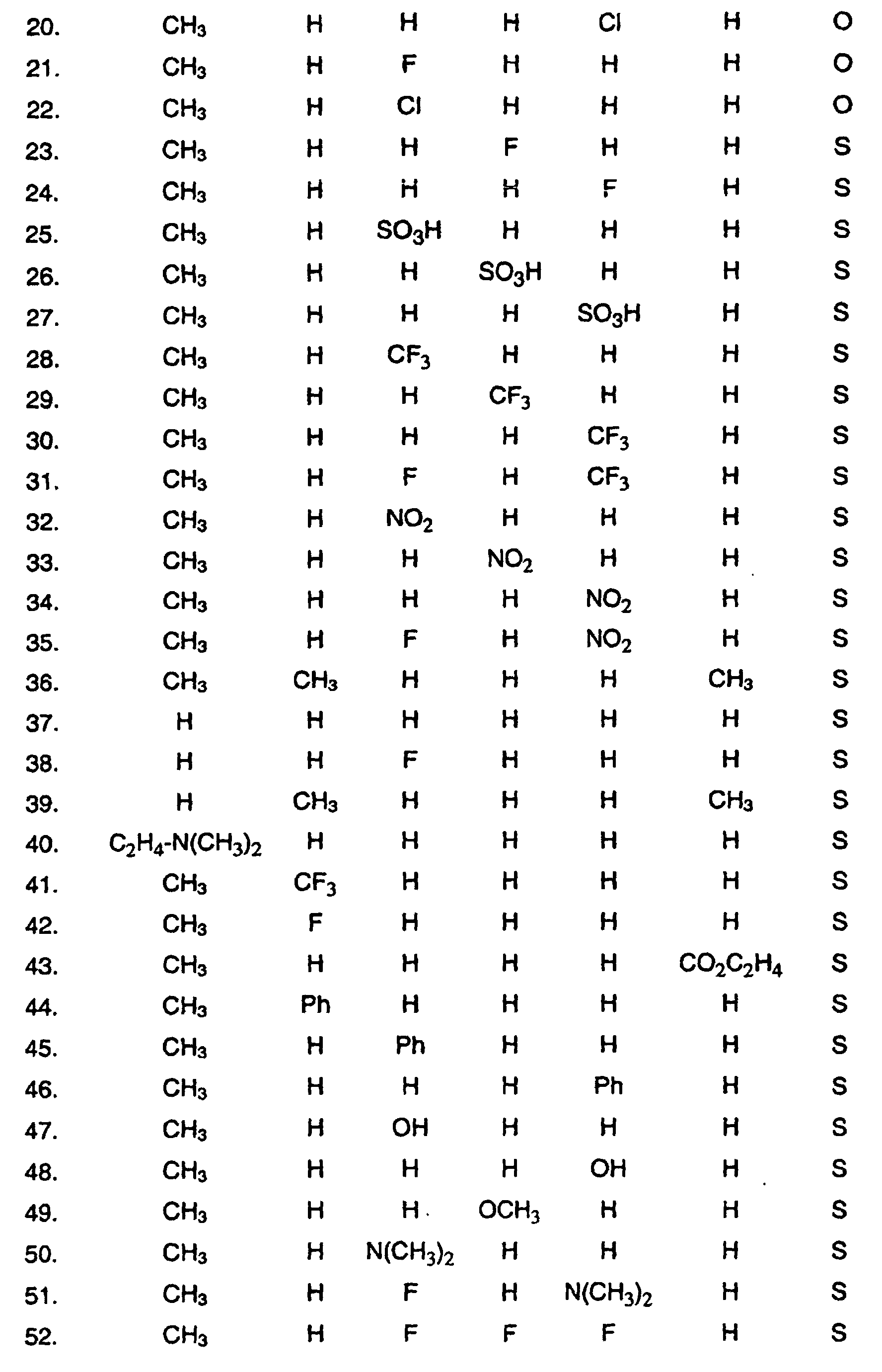

Wie gefunden wurde, zeichnen sich die Verbindungen der allgemeinen Formel (I) durch vielfältige Anwendungsmöglichkeiten auf therapeutischem Gebiet aus. Hervorzuheben sind solche Anwendungsmöglichkeiten, für welche die positive Modulation von AMPA Rezeptoren eine Rolle spielen.
Die Wirkung der erfindungsgemäßen Verbindungen als AMPA-Rezeptor-Modulatoren wurde elektrophysiologisch an Zellen gemessen, die funktionelle AMPA Rezeptoren expremieren. Dabei wurde untersucht, ob die Testsubstanzen den Agonisten-induzierten Strom positiv allosterisch beeinflussen.

Der Test erfolgte bei Konzentrationen zwischen 0.3 µmol und 300 µmol.

**Tabelle 2:**

| Verstärkung des durch Agonisten induzierten Stroms (+ gute, ++ sehr gute Wirkung) | |
|---|---|
| Beispiel | Wirksamkeit |
| 1 | ++ |
| 2 | + |
| 3 | + |
| 4 | ++ |
| 5 | + |
| 15 | + |

Auch lassen sich mit den neuen Verbindungen Krankheiten oder Zustände behandeln, bei denen neuronale Netzwerke, die für ihre Funktion AMPA Rezeptoren benötigen, geschädigt oder in ihrer Funktion beeinträchtigt werden.
Die Verbindungen der allgemeinen Formel (I) können so bei Demenzerkrankungen, bei neurodegenerativen oder psychotischen Erkrankungen und bei neurodegenerativen Störungen sowie Gehimischämien verschiedener Genese, bevorzugt bei Schizophrenie oder Lern- und Gedächtnisstörungen, eingesetzt werden.

Ferner fallen hierunter: Status epileptikus, Hypoglykämie, Hypoxie, Anoxie, Gehimtrauma, Gehimoedem, amyotrope laterale Sklerose, Huntington's Disease, Morbus Alzheimer, sexuelle Dysfunktion, Störungen der sensorisch/motorischen Funktion, Gedächtnisbildung, hyperkinetische Verhaltensänderungen (insbesondere bei Kindern), Hypotonie, Herzinfarkt, Himdruck (erhöhter intrakranialer Druck), ischämischer und hämorrhagischer Schlaganfall, globale cerebrale Ischämie bei Herzstillstand, akuter und chronischer neuropathischer Schmerz, Diabetische Polyneuropathie, Tinnitus, perinatale Asphyxie, Psychose, Morbus Parkinson und Depression, sowie damit verbundene Angstzustände.

Die neuen Verbindungen können auch in Kombination mit anderen Wirkstoffen angewendet werden, etwa solchen, die für dieselben Indikationen Verwendung finden, oder z.B. mit Neuroteptika, Nootropika, Psychostimulantien, etc. Die Verabreichung kann topisch, oral, transdermal, nasal, parenteral oder inhalativ erfolgen. Ferner können die Verbindungen der allgemeinen Formel I bzw. deren Salze auch mit andersartigen Wirkstoffen kombiniert werden.

Die Verbindungen der allgemeinen Formel (I) können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1 - 90 Gew.-%, bevorzugt 0,5 - 50 Gew.-% der Gesamtzusammensetzung liegen, d.h. in Mengen die ausreichend sind, um den unten angegebenen Dosierungsbereich zu erreichen.
Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbessemdes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägem, wie Milchzucker oder Sorbit, mischt und in Gelatinekapseln einkapselt.
Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserem oder Farbstoffen versetzt werden.
Für den Fall der parenteralen Anwendung können Lösungen der Wirkstoffe unter Verwendung geeigneter flüssiger Trägermaterialien eingesetzt werden.
Die Dosierung für die intravenöse Anwendung liegt bei 1 - 1000 mg pro Stunde, vorzugsweise zwischen 5 - 500 mg pro Stunde.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einxelgaben über der Tag zu verteilen.

Die nachfolgenden Formulierungsbeipiele illustrieren die vorliegende Erfindung ohne sie jedoch in ihrem Umfang zu beschränken:

### Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | | |
| | Wirkstoff | 50 mg |
| | Natriumchlorid | 50 mg |
| | Aqua pro inj. | 5 ml |

Der Wirkstoff wird bei Eigen-pH oder gegebenenfalls bei pH 5,5 - 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Verbindungen der allgemeinen Formel (I) 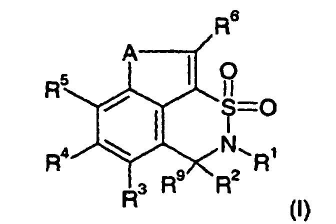 worin
A ein Schwefelatom, Sauerstoffatom, NH oder N-C₁-C₄―Alkyl,
R¹ ein Rest ausgewählt aus der Gruppe bestehend aus Wasserstoff, einer gegebenenfalls durch ein oder mehrere Halogenatome substituierten C₁-C₆-Alkylgruppe, -SO₂H, -SO₂-C₁-C₆-Alkyl, -SO-C₁-C₆-Alkyl, -CO-C₁-C₆-Alkyl, -O, Phenyl-C₁-C₄-Alkyl, -C₁-C₄-Alkyl-NR⁷R⁸ und -C₁-C₄―Alkyl-O- C₁-C₄―Alkyl, C₃-C₆-Cycloalkyl,
R², R⁹ gleich oder verschieden, ein Rest ausgewählt aus der Gruppe bestehend aus Wasserstoff, einer gegebenenfalls durch ein oder mehrere Halogenatome substituierten C₁-C₆-Alkylgruppe, Halogen, -NO₂, -SO₂H, -SO₂-C₁-C₆-Alkyl, -SO-C₁-C₆-Alkyl, -CO-C₁-C₆-Alkyl, -OH, -O-C₁-C₆-Alkyl, -S-C₁-C₆-Alkyl, -C₁-C₄-Alkyl-NR⁷R⁸ und -C₁-C₄-Alkyl-O- C₁-C₄-Alkyl, C₃-C₆-Cycloalkyl, oder
R¹ und R² gemeinsam eine C₂-C₆-Alkylenbrücke,
R⁷, R⁸ gleich oder verschieden, Wasserstoff oder C₁-C₄-Alkyl, und
R³, R⁴, R⁵, R⁶ gleich oder verschieden, ein Rest ausgewählt aus der Gruppe bestehend aus Wasserstoff, einer gegebenenfalls durch ein oder mehrere Halogenatome substituierten C₁-C₆-Alkyl Gruppe, Phenyl-C₁-C₄―Alkyl, Halogen, -CN, -NO₂, -SO₂H, -SO₃H, -SO₂-C₁-C₆-Alkyl, -SO-C₁-C₆-Alkyl, -SO₂-NR⁷R⁸, -COOH, -CO-C₁-C₆-Alkyl, -O-CO-C₁-C₄-Alkyl, -CO-O-C₁-C₄-Alkyl, -CO-NR⁷R⁸, -C₁-C₄-Alkyl, -OH, -O-C₁-C₆-Alkyl, -S-C₁-C₆-Alkyl, -NR⁷R⁸ und einem gegebenenfalls einfach oder mehrfach durch Halogenatome, -NO₂, -(SO₂H oder C₁-C₄-Alkyl substituierten Aryl-Rest, bedeuten,
wobei die in den Resten R¹ bis R⁹ genannten Alkylgruppen gegebenenfalls durch ein oder mehrere Halogenatome, ausgewählt aus der Gruppe bestehend aus Fluor, Chlor, Brom und lod, substituiert sein können, und
die in den Resten R¹ und R³ bis R⁶ genannten Arylgruppen gegebenenfalls durch Halogenatome, -NO₂, -SO₂H oder -C₁-C₄-Alkyl substituiert sein können.
gegebenenfalls in Form ihrer verschiedenen Enantiomeren und
Diastereomeren, sowie deren pharmakologisch unbedenklichen Salze.

2. Verbindungen der allgemeinen Formel (I) gemäß Anspruch 1, worin
A ein Schwefelatom, Sauerstoffatom oder N-C₁-C₂―Alkyl,
R¹ ein Rest ausgewählt aus der Gruppe bestehend aus Wasserstoff, einer gegebenenfalls durch ein oder mehrere Halogenatome substituierten C₁-C₆-Alkylgruppe, -SO₂H, -SO₂-C₁-C₆-Alkyl, -SO-C₁-C₆-Alkyl, -CO-C₁-C₆-Alkyl, -O, -C₁-C₄―Alkyl-NR⁷R⁸ und -C₁-C₄―Alkyl-O- C₁-C₄―Alkyl, Benzyl,
R², R⁹ gleich oder verschieden, ein Rest ausgewählt aus der Gruppe bestehend aus Wasserstoff, einer gegebenenfalls durch ein oder mehrere Halogenatome substituierten C₁-C₆-Alkylgruppe, Halogen, -NO₂, -SO₂H, -SO₂-C₁-C₆-Alkyl, -SO-C₁-C₆-Alkyl, -CO-C₁-C₆-Alkyl, -OH, -O-C₁-C₆-Alkyl, -S-C₁-C₆-Alkyl, -C₁-C₄-Alkyl-NR⁷R⁸ und -C₁-C₄―Alkyl-O- C₁-C₄-Alkyl, oder
R¹ und R² gemeinsam eine C₃-C₆-Alkylenbrücke, und
R³, R⁴, R⁵, R⁶ gleich oder verschieden, ein Rest ausgewählt aus der Gruppe bestehend aus Wasserstoff, einer gegebenenfalls durch ein oder mehrere Halogenatome substituierten C₁-C₄-Alkyl Gruppe, C₁-C₄-Alkylen-phenyl, Halogen, -CN, -NO₂, -SO₂H, -SO₃H, -SO₂CH₃, -SOCH₃, -CO-C₁-C₄-Alkyl, -OH, -O-C₁-C₄-Alkyl und -S-C₁-C₄-Alkyl bedeuten,
wobei die in A und in den Resten R¹ bis R⁶ und R⁹ genannten Alkylgruppen gegebenenfalls durch ein oder mehrere Halogenatome, ausgewählt aus der Gruppe bestehend aus Fluor, Chlor, Brom und lod, substituiert sein können, und
die in dem Rest R¹ genannte Benzylgruppe gegebenenfalls durch Halogenatome, -NO₂, -SO₂H oder -C₁-C₄-Alkyl substituiert sein kann. gegebenenfalls in Form ihrer verschiedenen Enantiomeren und Diastereomeren, sowie deren pharmakologisch unbedenklichen Salze.

3. Verbindungen der allgemeinen Formel (I) gemäß Anspruch 1 oder 2, worin
A ein Schwefelatom oder N-C₁-C₂―Alkyl,
R¹, R², R⁹ gleich oder verschieden, Wasserstoff, C₁-C₄-Alkyl, Benzyl oder
R¹ und R² gemeinsam eine C₃-C₄-Alkylenbrücke, und
R³, R⁴, R⁵, R⁶ gleich oder verschieden, ein Rest ausgewählt aus der Gruppe bestehend aus Wasserstoff, C₁-C₄-Alkyl, CF₃, NO₂, Benzyl, -SO₂-C₁-C₄-Alkyl, -SO₃H und Halogen bedeuten,
wobei die in A und den Resten R¹ bis R⁶ und R⁹ genannten Alkylgruppen gegebenenfalls durch ein oder mehrere Halogenatome, ausgewählt aus der Gruppe bestehend aus Fluor, Chlor, Brom und lod, substituiert sein können, und
die in den Resten R¹ bis R⁶ und R⁹ genannte Benzylgruppe gegebenenfalls durch Halogenatome, -NO₂, -SO₂H oder -C₁-C₄-Alkyl substituiert sein kann.
gegebenenfalls in Form ihrer verschiedenen Enantiomeren und
Diastereomeren, sowie deren pharmakologisch unbedenklichen Salze.

4. Verbindungen der allgemeinen Formel (I) gemäß Anspruch 1 bis 3, worin
A ein Schwefelatom oder N-CH₃,
R¹, R², R⁹ gleich oder verschieden, Wasserstoff, C₁-C₄-Alkyl oder
R¹ und R² gemeinsam eine C₃-C₄-Alkylenbrücke,
R³, R⁵, R⁶ gleich oder verschieden, ein Rest ausgewählt aus der Gruppe bestehend aus Wasserstoff, C₁-C₄-Alkyl und Halogen, und
R⁴ Wasserstoff, Halogen oder C₁-C₄-Alkyl bedeuten,
wobei die in den Resten R¹ bis R⁶ und R⁹ genannten Alkylgruppen gegebenenfalls durch ein oder mehrere Halogenatome, ausgewählt aus der Gruppe bestehend aus Fluor, Chlor, Brom und Iod, substituiert sein können,
gegebenenfalls in Form ihrer verschiedenen Enantiomeren und
Diastereomeren, sowie deren pharmakologisch unbedenklichen Salze.

5. Verbindung der allgemeinen Formel (I) gemäß einem der Ansprüche 1 bis 4, worin
R¹ Methyl, Ethyl, i-Propyl, n-Butyl oder Benzyl steht,
wobei die in dem Rest R¹ genannten Methyl, Ethyl, i-Propyl oder n-Butyl-Gruppen, gegebenenfalls durch ein oder mehrere Halogenatome, ausgewählt aus der Gruppe bestehend aus Fluor, Chlor, Brom und lod, substituiert sein können, und
die in dem Rest R¹ genannte Benzylgruppe gegebenenfalls durch Halogenatome, -NO₂, -SO₂H oder -C₁-C₄-Alkyl substituiert sein kann,
gegebenenfalls in Form ihrer verschiedenen Enantiomeren und
Diastereomeren, sowie deren pharmakologisch unbedenklichen Salze.

6. Verbindungen der allgemeinen Formel (I) gemäß Anspruch 1 bis 5, worin
A ein Schwefelatom,
R¹ Methyl,
R², R⁹ Wasserstoff,
R³ ein Rest ausgewählt aus der Gruppe bestehend aus Wasserstoff, Methyl, CN und Halogen,
R⁵ ein Rest ausgewählt aus der Gruppe bestehend aus Wasserstoff, Methyl und Halogen,
R⁴ Wasserstoff, und
R⁶ Wasserstoff oder Methyl bedeuten,
wobei die in den Resten R¹, R³, R⁵ und R⁶ genannten Methylgruppen gegebenenfalls durch ein oder mehrere Halogenatome, ausgewählt aus der Gruppe bestehend aus Fluor, Chlor, Brom und lod, substituiert sein können,
gegebenenfalls in Form ihrer pharmakologisch unbedenklichen Salze.

7. Verbindungen der allgemeinen Formel (I) gemäß Anspruch 1, worin
A ein Schwefelatom,
R¹ gegebenenfalls durch ein oder mehrere Halogenatome, ausgewählt aus der Gruppe bestehend aus Fluor, Chlor, Brom und lod substituiertes Methyl,
R³ Wasserstoff, Fluor oder Chlor, und
R^{2,}, R⁴, R⁵, R⁶, R⁹ Wasserstoff bedeuten,
gegebenenfalls in Form ihrer pharmakologisch unbedenklichen Salze

8. Verbindung der allgemeinen Formel (I) gemäß einem der Ansprüche 1 bis 7 zur Verwendung als Arzneimittel.

9. Verwendung einer Verbindung der allgemeinen Formel (I) gemäß Anspruch 8 als Arzneimittel.

10. Verbindung der allgemeinen Formel (I) gemäß Anspruch 8 zur Verwendung als Arzneimittel mit neuroprotektiver Wirkung.

11. Verwendung einer Verbindung der allgemeinen Formel (I) gemäß Anspruch 10 als Arzneimittel mit neuroprotektiver Wirkung.

12. Verwendung einer Verbindung der allgemeinen Formel (I) gemäß einem der Ansprüche 1 bis 7 zur Herstellung eines Arzneimittels zur Behandlung und/oder Prävention von neurodegenerativen Erkrankungen und/oder Gehimischämie verschiedener Genese.

13. Verwendung einer Verbindung der allgemeinen Formel (I) gemäß einem der Ansprüche 1 bis 7 zur Herstellung eines Arzneimittels zur Behandlung von Schizophrenie.

14. Verwendung einer Verbindung der allgemeinen Formel (I) gemäß einem der Ansprüche 1 bis 7 zur Herstellung eines Arzneimittels zur Behandlung und/oder Prävention von Gedächtnisstörungen.

15. Verwendung einer Verbindung der allgemeinen Formel (I) gemäß einem der Ansprüche 1 bis 7 zur Herstellung eines Arzneimittels zur Behandlung von Demenzerkrankungen.

16. Pharmazeutische Zubereitungen, enthaltend als Wirkstoff eine oder mehrere Verbindungen der allgemeinen Formel (I) gemäß einem der Ansprüche 1 bis 7 oder deren physiologisch verträgliche Salze gegebenenfalls in Kombination mit üblichen Hilfs- und/oder Trägerstoffen.

## Claims

1. Compounds of general formula (I) 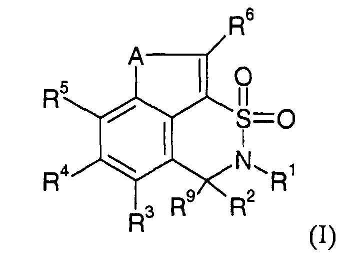 wherein
A denotes a sulphur atom, oxygen atom, NH or N-C₁-C₄―alkyl,
R¹ denotes a group selected from among hydrogen, a C₁-C₆-alkyl group optionally substituted by one or more halogen atoms, -SO₂H, -SO₂-C₁-C₆-alkyl, -SO-C₁-C₆-alkyl, -CO-C₁-C₆-alkyl, -O, phenyl-C₁₋C₄-alkyl, -C₁-C₄-alkyl-NR⁷R⁸ and -C₁-C₄-alkyl-O- C₁-C₄―alkyl, C₃-C₆-cycloalkyl,
R², R⁹, which may be identical or different, denote a group selected from among hydrogen, a C₁-C₆-alkyl group optionally substituted by one or more halogen atoms, halogen, -NO₂, -SO₂H, -SO₂-C₁-C₆-alkyl, -SO-C₁-C₆-alkyl, -CO-C₁-C₆-alkyl, -OH, -O-C₁-C₆-alkyl, -S-C₁-C₆-alkyl, -C₁-C₄-alkyl-NR⁷R⁸ and -C₁-C₄-alkyl-O- C₁-C₄-alkyl, C₃-C₆-cycloalkyl, or
R¹ and R² together denote a C₂-C₆-alkylene bridge,
R⁷ , R⁸ , which may be identical or different, denote hydrogen or C₁-C₄-alkyl, and
R³, R⁴, R⁵, R⁶ , which may be identical or different, denote a group selected from among hydrogen, a C₁-C₆-alkyl group optionally substituted by one or more halogen atoms, phenyl-C₁-C₄-alkyl, halogen, -CN, -NO₂, -SO₂H, -SO₃H, -SO₂-C₁-C₆-alkyl, -SO-C₁-C₆-alkyl, -SO₂-NR⁷R⁸, -COOH, -CO-C₁-C₆-alkyl, -O-CO-C₁-C₄-alkyl, -CO-O-C₁-C₄-alkyl, -CO-NR⁷R⁸-C₁-C₄-alkyl, -OR, -O-C₁-C₆-alkyl, -S-C₁-C₆-alkyl, -NR⁷R⁸ and an aryl group optionally mono- or polysubstituted by halogen atoms, -NO₂, -SO₂H or C₁-C₄-alkyl,
while the alkyl groups mentioned in groups R¹ to R⁹ may optionally be substituted by one or more halogen atoms selected from the group consisting of fluorine, chlorine, bromine and iodine, and
the aryl groups mentioned in groups R¹ and R³ to R⁶ may optionally be substituted by halogen atoms, -NO₂, -SO₂H or -C₁-C₄-alkyl,
optionally in the form of the various enantiomers and diastereomers thereof, as well as the pharmacologically acceptable salts thereof.

2. Compounds of general formula (I) according to claim 1, wherein
A denotes a sulphur atom, oxygen atom or N-C₁-C₂-alkyl,
R¹ denotes a group selected from among hydrogen, a C₁-C₆-alkyl group optionally substituted by one or more halogen atoms, -SO₂H, -SO₂-C₁-C₆-alkyl, -SO-C₁-C₆-alkyl, -CO-C₁-C₆-alkyl, -O, -C₁-C₄-alkyl-NR⁷R⁸ and -C₁-C₄-alkyl-O-C₁-C₄-alkyl, benzyl,
R², R⁹ , which may be identical or different, denote a group selected from among hydrogen, a C₁-C₆-alkyl group optionally substituted by one or more halogen atoms, halogen, -NO₂, -SO₂H, -SO₂-C₁-C₆-alkyl, -SO-C₁-C₆-alkyl, -CO-C₁-C₆-alkyl, -OH, -O-C₁-C₆-alkyl, -S-C₁-C₆-alkyl, -C₁-C₄-alkyl-NR⁷R⁸ and -C₁-C₄―alkyl-O- C₁-C₄-alkyl, or
R¹ and R² together denote a C₃-C₆-alkylene bridge, and
R³, R⁴, R⁵, R⁶, which may be identical or different, denote a group
selected from among hydrogen, a C₁-C₄-alkyl group optionally substituted by one or more halogen atoms, C₁-C₄-alkylene- phenyl, halogen, -CN,
-NO₂, -SO₂H, -SO₃H, -SO₂CH₃, -SOCH₃, -CO-C₁-C₄-alkyl, -OH, -O-C₁-C₄-alkyl and -S-C₁-C₄-alkyl,
while the alkyl groups mentioned in A and in groups R¹ to R⁶ and R⁹ may optionally be substituted by one or more halogen atoms selected from the group consisting of fluorine, chlorine, bromine and iodine, and
the benzyl group mentioned in the group R' may optionally be substituted by halogen atoms, -NO₂, -SO₂H or -C₁-C₄-alkyl,
optionally in the form of the various enantiomers and diastereomers thereof, as well as the pharmacologically acceptable salts thereof.

3. Compounds of general formula (I) according to claim 1 or 2, wherein
A denotes a sulphur atom or N-C₁-C₂-alkyl,
R¹, R², R⁹, which may be identical or different, denote hydrogen, C₁-C₄-alkyl or benzyl or
R¹ and R² together denote a C₃-C₄-alkylene bridge, and
R³, R⁴, R⁵, R⁶, which may be identical or different, denote a group selected from among hydrogen, C₁-C₄-alkyl, CF₃, NO₂, benzyl, -SO₂-C₁-C₄-alkyl, -SO₃H and halogen,
while the alkyl groups mentioned in A and in groups R¹ to R⁶ and R⁹ may optionally be substituted by one or more halogen atoms selected from the group consisting of fluorine, chlorine, bromine and iodine, and
the benzyl group mentioned in the groups R¹ to R⁶ and R⁹ may optionally be substituted by halogen atoms, -NO₂, -SO₂H or -C₁-C₄-alkyl,
optionally in the form of the various enantiomers and diastereomers thereof, as well as the pharmacologically acceptable salts thereof.

4. Compounds of general formula (I) according to claims 1 to 3, wherein
A denotes a sulphur atom or N-CH₃,
R¹, R², R⁹, which may be identical or different, denote hydrogen or C₁-C₄-alkyl or
R¹ and R² together denote a C₃-C₄-alkylene bridge,
R³, R⁵, R⁶, which may be identical or different, denote a group selected from among hydrogen, C₁-C₄-alkyl and halogen, and
R⁴ denotes hydrogen, halogen or C₁-C₄-alkyl,
while the alkyl groups mentioned in groups R¹ to R⁶ and R⁹ may optionally be substituted by one or more halogen atoms selected from the group consisting of fluorine, chlorine, bromine and iodine,
optionally in the form of the various enantiomers and diastereomers thereof, as well as the pharmacologically acceptable salts thereof.

5. Compound of general formula (I) according to one of claims 1 to 4, wherein
R¹ denotes methyl, ethyl, i-propyl, n-butyl or benzyl,
while the methyl, ethyl, i-propyl or n-butyl groups mentioned in the group R¹ may optionally be substituted by one or more halogen atoms selected from the group consisting of fluorine, chlorine, bromine and iodine, and
the benzyl group mentioned in the group R¹ may optionally be substituted by halogen atoms, -NO₂, -SO₂H or -C₁-C₄-alkyl,
optionally in the form of the various enantiomers and diastereomers thereof, as well as the pharmacologically acceptable salts thereof.

6. Compounds of general formula (I) according to claims 1 to 5, wherein
A denotes a sulphur atom,
R¹ denotes methyl,
R², R⁹ denote hydrogen,
R³ denotes a group selected from among hydrogen, methyl, CN and halogen,
R⁵ denotes a group selected from among hydrogen, methyl and halogen,
R⁴ denotes hydrogen, and
R⁶ denotes hydrogen or methyl,
while the methyl groups mentioned in the groups R¹, R³, R⁵ and R⁶ may optionally be substituted by one or more halogen atoms selected from the group consisting of fluorine, chlorine, bromine and iodine,
optionally in the form of the pharmacologically acceptable salts thereof.

7. Compounds of general formula (I) according to claim 1, wherein
A denotes a sulphur atom,
R¹ denotes methyl, optionally substituted by one or more halogen atoms selected from the group consisting of fluorine, chlorine, bromine and iodine,
R³ denotes hydrogen, fluorine or chlorine, and
R² , R⁴, R⁵, R⁶, R⁹ denote hydrogen,
optionally in the form of the pharmacologically acceptable salts thereof

8. Compound of general formula (I) according to one of claims 1 to 7 for use as a pharmaceutical composition.

9. Use of a compound of general formula (I) according to claim 8 as a pharmaceutical composition.

10. Compound of general formula (I) according to claim 8 for use as a pharmaceutical composition having a neuroprotective effect.

11. Use of a compound of general formula (I) according to claim 10 as a pharmaceutical composition having a neuroprotective effect.

12. Use of a compound of general formula (I) according to one of claims 1 to 7 for preparing a pharmaceutical composition for the treatment and/or prevention of neurodegenerative diseases and/or cerebral ischaemia of various origins.

13. Use of a compound of general formula (I) according to one of claims 1 to 7 for preparing a pharmaceutical composition for the treatment of schizophrenia.

14. Use of a compound of general formula (I) according to one of claims 1 to 7 for preparing a pharmaceutical composition for the treatment and/or prevention of memory disorders.

15. Use of a compound of general formula (I) according to one of claims 1 to 7 for preparing a pharmaceutical composition for the treatment of dementias.

16. Pharmaceutical preparations containing as active substance one or more compounds of general formula (I) according to one of claims 1 to 7 or the physiologically acceptable salts thereof, optionally combined with conventional excipients and/or carriers.

## Revendications

1. Composés de formule générale (I) 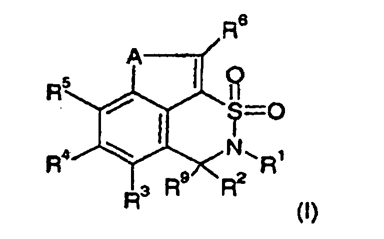 dans laquelle
A représente un atome de soufre, un atome d'oxygène, NH ou N-(alkyle en C₁ à C₄),
R¹ représente un groupement choisi dans le groupe constitué de l'hydrogène, un groupe alkyle en C₁ à C₆ éventuellement substitué par un ou plusieurs atomes d'halogène, -SO₂H, -SO₂-(alkyle en C₁ à C₆), -SO-(alkyle en C₁ à C₆), -CO-(alkyle en C₁ à C₆), -O, phényl-(alkyle en C₁ à C₄), -(alkyle en C₁ à C₄)-NR⁷R⁸ et -(alkyle en C₁ à C₄)-O-(alkyle en C₁ à C₄), cycloalkyle en C₃ à C₆,
R², R⁹ identiques ou différents, représentent un groupement choisi dans le groupe constitué de l'hydrogène, un groupe alkyle en C₁ à C₆ éventuellement substitué par un ou plusieurs atomes d'halogène, halogène, -NO₂, -SO₂H, -SO₂-(alkyle en C₁ à C₆), -SO-(alkyle en C₁ à C₆), -CO-(alkyle en C₁ à C₆), -OH, -O-(alkyle en C₁ à C₆)-S-(alkyle en C₁ à C₆), -(alkyle en C₁ à C₄)-NR⁷R⁸ et -(alkyle en C₁ à C₄)-O-(alkyle en C₁ à C₄), cycloalkyle en C₃ à C₆,
ou
R¹ et R² forment ensemble un pont alkylène en C₂ à C₆,
R⁷, R⁸ identiques ou différents, représentent un hydrogène ou alkyle en C₁ à C₄, et R³, R⁴, R⁵, R⁶ identiques ou différents, représentent un groupement choisi dans le groupe constitué de l'hydrogène, un groupe alkyle en C₁ à C₆ éventuellement substitué par un ou plusieurs atomes d'halogène, phényl-(alkyle en C₁ à C₄), un halogène, -CN, -NO₂, -SO₂H, -SO₃H, -SO₂-(alkyle en C₁ à C₆), -SO-(alkyle en C₁ à C₆), -SO₂-NR⁷R⁸, -COOH, -CO-(alkyle en C₁ à C₆), -O-CO-(alkyle en C₁ à C₄), -CO-O-(alkyle en C₁ à C₄), -CO-NR⁷R⁸ -(alkyle en C₁ à C₄), -OH, -O-(alkyle en C₁ à C₆), -S-(alkyle en C₁ à C₆), -NR⁷R⁸ et un groupement aryle éventuellement substitué une ou plusieurs fois par des atomes d'halogène, -NO₂, -SO₂H ou alkyle en C₁ à C₄,
dans lesquels les groupes alkyle mentionnés dans les groupements R¹ à R⁹ peuvent éventuellement être substitués par un ou plusieurs atomes d'halogène, choisis dans le groupe constitué du fluor, chlore, brome et de l'iode, et les groupes aryle mentionnés dans les groupements R¹ et R³ à R⁶ peuvent éventuellement être substitués par des atomes d'halogène, -NO₂, -SO₂H ou alkyle en C₁ à C₄,
éventuellement sous la forme de leurs différents énantiomères et diastéréomères, ainsi que de leurs sels pharmaceutiquement acceptables.

2. Composés de formule générale (I) selon la revendication 1, dans lesquels
A représente un atome de soufre, un atome d'oxygène ou N-(alkyle en C₁ à C₂),
R¹ représente un groupement choisi dans le groupe constitué de l'hydrogène, d'un groupe alkyle en C₁ à C₆ éventuellement substitué par un ou plusieurs atomes d'halogène, -SO₂H, -SO₂-(alkyle en C₁ à C₆), -SO-(alkyle en C₁ à C₆), -CO-(alkyle en C₁ à C₆), -O, -(alkyle en C₁ à C₄)-NR⁷R⁸ et -(alkyle en C₁ à C₄)-O-(alkyle en C₁ à C₄), benzyle,
R², R⁹ identiques ou différents, représentent un groupement choisi dans le groupe constitué de l'hydrogène, d'un groupe alkyle en C₁ à C₆ éventuellement substitué par un ou plusieurs atomes d'halogène, halogène, -NO₂, -SO₂H, -SO₂-(alkyle en C₁ à C₆), -SO-(alkyle en C₁ à C₆), -CO-(alkyle en C₁ à C₆), -OH, -O-(alkyle en C₁ à C₆), -S-(alkyle en C₁ à C₆), -(alkyle en C₁ à C₄)-NR⁷R⁸ et -(alkyle en C₁ à C₄)-O-(alkyle en C₁ à C₄), ou R¹ et R² forment ensemble un pont alkylène en C₃ à C₆,
R³, R⁴, R⁵, R⁶ identiques ou différents, représentent un groupement choisi dans le groupe constitué de l'hydrogène, un groupe alkyle en C₁ à C₄ éventuellement substitué par un ou plusieurs atomes d'halogène, (alkylène en C₁ à C₄)-phényle, un halogène, -CN, -NO₂, -SO₂H, -SO₃H, -SO₂CH₃, -SOCH₃, -CO-(alkyle en C₁ à C₄), -OH, -O-(alkyle en C₁ à C₄) et -S-(alkyle en C₁ à C₄),
dans lesquels les groupes alkyle mentionnés dans A et dans les groupements R¹ à R⁶ et R⁹ peuvent éventuellement être substitués par un ou plusieurs atomes d'halogène, choisis dans le groupe constitué du fluor, chlore, brome et de l'iode, et
le groupe benzyle mentionné dans le groupement R' peut éventuellement être substitué par des atomes d'halogène, -NO₂, -SO₂H ou alkyle en C₁ à C₄.
éventuellement sous la forme de leurs différents énantiomères et diastéréomères, ainsi que de leurs sels pharmaceutiquement acceptables.

3. Composés de formule générale (I) selon la revendication 1 ou 2, dans lesquels
A représente un atome de soufre ou N-alkyle en C₁ à C₂,
R¹, R², R⁹ identiques ou différents, représentent un hydrogène, alkyle en C₁ à C₄, benzyle ou
R¹ et R² forment ensemble un pont alkylène en C₃ à C₄, et
R³, R⁴, R⁵, R⁶ identiques ou différents, représentent un groupement choisi dans le groupe constitué de l'hydrogène, un alkyle en C₁ à C₄, CF₃, NO₂, benzyle, -SO₂-(alkyle en C₁ à C₄), -SO₃H et un halogène,
dans lesquels les groupes alkyle mentionnés dans A et dans les groupements R¹ à R⁶ et R⁹ peuvent éventuellement être substitués par un ou plusieurs atomes d'halogène, choisis dans le groupe constitué du fluor, chlore, brome et de l'iode, et
le groupe benzyle mentionné dans les groupements R¹ à R⁶ et R⁹ peut éventuellement être substitué par des atomes d'halogène, -NO₂, -SO₂H ou alkyle en C₁ à C₄,
éventuellement sous la forme de leurs différents énantiomères et diastéréomères, ainsi que de leurs sels pharmaceutiquement acceptables.

4. Composés de formule générale (I) selon les revendications 1 à 3, dans lesquels
A représente un atome de soufre ou N-CH₃,
R¹, R², R⁹ identiques ou différents, représentent un hydrogène, alkyle en C₁ à C₄ ou
R¹ et R² forment ensemble un pont alkylène en C₃ à C₄,
R³, R⁵, R⁶ identiques ou différents, représentent un groupement choisi dans le groupe constitué de l'hydrogène, un alkyle en C₁ à C₄ et un halogène, et
R⁴ représente un hydrogène, halogène ou alkyle en C₁ à C₄,
dans lesquels les groupes alkyle mentionnés dans les groupements R¹ à R⁶ et R⁹ peuvent éventuellement être substitués par un ou plusieurs atomes d'halogène, choisis dans le groupe constitué du fluor, chlore, brome et de l'iode,
présents éventuellement sous la forme de leurs différents énantiomères et diastéréomères, ainsi que leurs sels pharmaceutiquement acceptables.

5. Composé de formule générale (I) selon une des revendications 1 à 4, dans lequel
R¹ représente un méthyle, éthyle, i-propyle, n-butyle ou benzyle,
dans lequel les groupes méthyle, éthyle, i-propyle ou n-butyle mentionnés dans le groupement R¹ peuvent éventuellement être substitués par un ou plusieurs atomes d'halogène, choisis dans le groupe constitué du fluor, chlore, brome et de l'iode, et
le groupe benzyle mentionné dans le groupement R' peut éventuellement être substitué par des atomes d'halogène, -NO₂, -SO₂H ou alkyle en C₁ à C₄,
éventuellement sous la forme de ses différents énantiomères et diastéréomères, ainsi que de ses sels pharmaceutiquement acceptables.

6. Composés de formule générale (I) selon les revendications 1 à 5, dans lesquels
A représente un atome de soufre,
R¹ représente un méthyle
R², R⁹ représentent un hydrogène,
R³ représente un groupement choisi dans le groupe constitué de l'hydrogène, méthyle, CN et halogène,
R⁵ représente un groupement choisi dans le groupe constitué de l'hydrogène, méthyle et halogène,
R⁴ représente un hydrogène, et
R⁶ représente un hydrogène ou méthyle,
dans lequel les groupes méthyle mentionnés dans les groupements R¹, R³, R⁵ et R⁶ peuvent éventuellement être substitués par un ou plusieurs atomes d'halogène, choisis dans le groupe constitué du fluor, chlore, brome et de l'iode,
éventuellement sous la forme de leurs différents énantiomères et diastéréomères, ainsi que leurs sels pharmaceutiquement acceptables.

7. Composés de formule générale (I) selon la revendication 1, dans lesquels
A représente un atome de soufre,
R¹ représente un méthyle éventuellement substitué par un ou plusieurs atomes d'halogène, choisis dans le groupe constitué du fluor, chlore, brome et de l'iode,
R³ représente un hydrogène, fluor ou chlore,
R², R⁴, R⁵, R⁶, R⁹ représentent un hydrogène,
éventuellement sous la forme de leurs sels pharmaceutiquement acceptables.

8. Composé de formule générale (I) selon une des revendications 1 à 7 destiné à une utilisation comme médicament.

9. Utilisation d'un composé de formule générale (I) selon la revendication 8 comme médicament.

10. Composé de formule générale (I) selon la revendication 8 destiné à une utilisation comme médicament ayant un effet neuroprotecteur.

11. Utilisation d'un composé de formule générale (I) selon la revendication 10 comme médicament ayant un effet neuroprotecteur.

12. Utilisation d'un composé de formule générale (I) selon une des revendications 1 à 7 pour la préparation d'un médicament destiné au traitement et/ou à la prévention de maladies neurodégénératives et/ou d'ischémie cérébrale de différentes origines.

13. Utilisation d'un composé de formule générale (I) selon l'une des revendications 1 à 7 pour la préparation d'un médicament destiné au traitement de la schizophrénie.

14. Utilisation d'un composé de formule générale (I) selon l'une des revendications 1 à 7 pour la préparation d'un médicament destiné au traitement et/ou la prévention de troubles de la mémoire.

15. Utilisation d'un composé de formule générale (I) selon l'une des revendications 1 à 7 pour la préparation d'un médicament destiné au traitement de démences.

16. Compositions pharmaceutiques, contenant comme substance active un ou plusieurs composés de formule générale (I) selon l'une des revendications 1 à 7 ou leurs sels physiologiquement acceptables éventuellement en combinaison avec des adjuvants et/ou véhicules usuels.
